# EUROPEAN PATENT SPECIFICATION

(11) **EP 1 663 928 B1**
(45) Date of publication and mention of the grant of the patent: **12.09.2007**
(21) Application number: 04741964.3
(22) Date of filing: 06.07.2004
(51) Int. Cl.: C07C 41/00, C08L 71/00, B01J 27/00

(54) **PREPARATION OF AN ALKOXYLATE COMPOSITION**
HERSTELLUNG EINER ALKOXYLATZUSAMMENSETZUNG
PREPARATION D'UNE COMPOSITION D'ALCOXYLATE

(30) Priority: 08.07.2003 US 485429 P
(43) Date of publication of application: 07.06.2006
(73) Proprietor: SHELL INTERNATIONALE RESEARCH MAATSCHAPPIJ B.V., 2596 HR Den Haag (NL)
(72) Inventor: ELEVELD, Michiel, Barend, NL-1031 CM Amsterdam (NL); MEURS, Jan, Hermen, Hendrik, NL-1031 CM Amsterdam (NL); RANEY, Kirk, Herbert, Houston, Texas 77094 (US); WIERSMA, Rendert, Jan, NL-1031 CM Amsterdam (NL); VAN ZON, Arie, NL-1031 CM Amsterdam (NL)
(86) International application number: PCT/EP2004/051366
(87) International publication number: WO 2005/005360

(56) References cited:
- EP-A- 0 589 635
- EP-A- 0 750 001
- WO-A-01/04183
- WO-A-02/42356
- WO-A-93/24549
- WO-A-03/091190
- WO-A-03/091191
- DE-A- 19 840 846
- US-A- 5 990 232
- US-B1- 6 391 820

## Description

The present invention relates to the use of a double metal cyanide catalyst for the preparation of an alkoxylate composition.

### Background of the Invention

A large variety of products useful, for instance, as nonionic surfactants, wetting and emulsifying agents, solvent, and chemical intermediates, are prepared by the addition reaction (alkoxylation reaction) of alkylene oxides (epoxides) with organic compounds having one or more active hydrogen atoms. For example, particular mention may be made of the alkanol ethoxylates and alkyl-substituted phenol ethoxylates prepared by the reaction of ethylene oxide with aliphatic alcohols or substituted phenols of 6 to 30 carbon atoms. Such ethoxylates, and to a lesser extent corresponding propoxylates and compounds containing mixed oxyethylene and oxypropylene groups, are widely employed as nonionic detergent components of commercial cleaning formulations for use in industry and in the home. As another example, the addition reaction of propylene oxide with polyols provides intermediates for the preparation of polyurethane products.

An illustration of the preparation of an alkanol ethoxylate (represented by formula III below) by addition of a number (k) of ethylene oxide molecules (formula II) to a single alkanol molecule (formula I) is presented by the equation

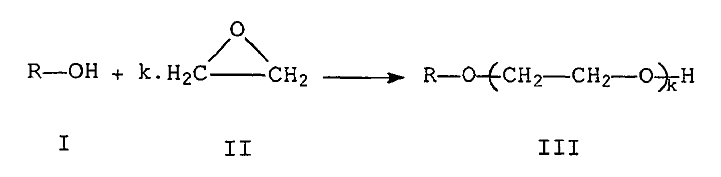

The term "alkoxylate", as used herein, refers to any product of the addition reaction of a number (k) of alkylene oxide molecules to a single active hydrogen containing organic compound.

Alkylene oxide addition reactions are known to produce a product mixture of various alkoxylate molecules having different numbers of alkylene oxide adducts (oxyalkylene adducts), e.g. having different values for the adduct number k in formula III above. The adduct number is a factor which in many respects controls the properties of the alkoxylate molecule, and efforts are made to tailor the average adduct number of a product and/or the distribution of adduct numbers within a product to the product's intended service.

It is known that alkoxylated alcohol products having a narrow range alkylene oxide adduct distribution are preferred for use in certain detergent formulations (GB-A-1462134; Research Disclosure No. 194010). Narrow-range alkoxylated alcohols are also known to be particularly valuable as chemical intermediates in the synthesis of certain carboxylated alkyl polyethers (US-A-4098818) and of certain alkyl ether sulfates (GB-A-1553561).

A commonly used class of alkoxylation catalysts are basic metal hydroxide compounds such as potassium hydroxide. Unfortunately, potassium hydroxide catalysts tend to suffer from the disadvantage that they give broader range alkylene oxide adducts than are desirable for many applications. As described above, for use in detergent formulations, it is preferred to provide alkoxylated alcohol products having a narrow range alkylene oxide adduct distribution.

In the preparation of alkoxylated alcohols, potassium hydroxide catalysts can also suffer from the disadvantage that they tend to produce alkoxylated alcohols containing high levels, typically of the order of 2% or above, of free alcohol. High levels of free alcohol are undesirable from the viewpoint of causing malodour. This is not ideal when the alkoxylated alcohol is being incorporated into household laundry detergents.

Attempts have been made to prepare alkoxylated alcohols having narrow range alkylene oxide adduct distribution.

For example, acidic catalysts such as Lewis acid and Bronsted acid catalysts are known for use as narrow range alkoxylation catalysts. However, although these acid catalysts provide a relatively narrow range alkylene oxide adduct distribution and relatively low levels of free alcohol, such catalysts suffer from the disadvantage that the alkoxylated alcohol products contain relatively high levels of 1,4-dioxanes as unwanted by-products. Again, this is less than ideal if the alkoxylated alcohol products are being included in a household laundry detergent.

Further, US-A-5057627 and WO02/047817 describe alkoxylation processes catalysed by phosphate salts of the rare earth elements. These catalysts are typically prepared by adding an aqueous solution of a rare earth compound such as lanthanum chloride to an aqueous sodium orthophosphate or H₃PO₄ solution. Although rare earth phosphates provide alkoxylated alcohol products having a narrow range alkylene oxide distribution, they have a tendency for producing relatively high levels of free alcohol.

In view of the above, it would be desirable to prepare an alkoxylate composition having a reduced level of free alcohol and preferably also a reduced level of 1,4-dioxanes.

It would be also desirable to prepare an alkoxylate composition having a reduced level of free alcohol and/or a reduced level of 1,4-dioxanes at the same time as having a narrow range alkylene oxide adduct distribution.

Double metal cyanide (DMC) compounds are well known catalysts for epoxide polymerization, i.e. for polymerizing alkylene oxides like propylene oxide and ethylene oxide to yield poly(alkylene oxide) polymers, also referred to as polyether polyols. Conventional DMC catalysts are prepared by reacting aqueous solutions of metal salts and metal cyanide salts to form a precipitate of the DMC compound.

WO01/04183 discloses polymerization of ethylene oxide using metal cyanide catalysts. Initiator compounds which have at least one oxyalkylatable group are mentioned as suitable initiator compounds for preparing poly(oxyethylene) polymers. However there is no mention of reduced levels of free active hydrogen containing organic compound or reduced levels of 1,4-dioxane in the final product.

WO02/42356 relates to the use of DMC catalysts for preparing polyether alcohols. Again, however, there is no mention in this document of reduced levels of free active hydrogen containing organic compound or reduced levels of 1,4-dioxane.

WO 03/091191 describes a composition comprising an alkoylate that may be made using a double metal cyanide catalyst.

WO 03/091190 also relates to an alkoxylate mixture which may be produced by the reaction of an alcohol with an alkylene oxide in the presence of a double metal cyanide catalyst.

DE 198 40 846 A1 describes a process for the preparation of unsupported and supported double metal cyanide catalysts and the use of said catalysts for the alkoxylation of fatty alcohols.

US 6,391,820 B1 relates to double metal cyanide catalysts comprising a) a double metal cyanide cyanide compound, b) an organic complex ligand other than c), and c) a glycoside, and the use of said double metal cyanide catalysts for the preparation of polyether polyols.

US 5,990,232 A describes polyethers containing both hydroxyl-functionality and unsaturation-functionality which are prepared by oxyalkylation of an unsaturated monomer having at least two free carboxylic acid groups in the presence of a DMC catalyst and optionally a free radical polymerization inhibitor. The resulting polyethers are suitable for use as polymer polyol stabilizers or stabilizer precursors, and both in situ and ex-situ impact modifiers for thermoplastics.

EP 0 750 001 A2 describes a process for the preparation of an ethylene oxide-capped polyol comprising: (a) blending a first polyether polyol, that contains an active double metal cyanide catalyst, with a second polyether polyol, that contains a basic catalyst; and (b) reacting the resulting polyol blend, containing 0.05 to 2 wt.% of the basic catalyst, with ethylene oxide to produce the ethylene oxide-capped polyol.

WO 93/24549 relates to a method for the fabrication of a thermoplastic elastomer which comprises the steps of: (a) preparing a polyol blend comprising a first polyol and a second polyol prepared using a DMC catalyst, a diisocyanate, and a difunctional, isocyanato-reactive chain-extender, wherein the first polyol has a molecular weight of between 1,000 and 5,000, said first polyol having an end group unsaturation level of no greater than 0.04 milliequivalents per gram of polyol, and the second polyol being a polyether polyol having an average molecular weight of between 1,000 and 20,000, (b) reacting said polyol blend with a diisocyanate to produce an isocyanate-terminated prepolymer, and (c) reacting said isocyanate-terminated prepolymer with a difunctional isocyanato-reactive chain extender in a mold or in an extruder in order to produce a hard elastomer characterized by a hardness of between a 75 Shore A and about a 75 Shore D. The polyols used therein are prepared by an alkylene oxide-polyhydric intitiator condensation reaction carried out in the presence of a double metal cyanide catalyst.

EP 0 589 635 A2 describes a process for removing DMC catalyst residues from a polyether polyol composition by adding a C₁ - C₆ aliphatic alcohol and a chelating agent, typically EDTA, to form an insoluble complex with the catalyst residues, followed by removal of the insoluble complex by filtration.

It has now surprisingly been found that by using a DMC catalyst for the alkoxylation of active hydrogen containing organic compounds, the alkoxylate product has reduced levels of free alcohol and/or reduced levels of 1,4-dioxane together with a narrow range alkylene oxide adduct distribution.

### Summary of the Invention

According to the present invention there is provided the use of a double metal cyanide complex catalyst for reducing the level of free alcohol in an alkoxylate composition prepared by the reaction of an alcohol with an alkylene oxide.

According to another aspect of the present invention there is provided the use of a double metal cyanide complex catalyst for reducing the level of 1,4-dioxane in an alkoxylate composition prepared by the reaction of an alcohol with an alkylene oxide.

### Detailed Description of the Invention

As used herein the term "reducing the level of free alcohol" means that the weight of free alcohol in the alkoxylate composition prepared using a DMC catalyst is no more than 60%, preferably no more than 40%, even more preferably no more than 20%, of the level of free alcohol in an equivalent alkoxylate composition prepared using a conventional potassium hydroxide catalyst. Typically, this means that the amount of free alcohol in the alkoxylate composition prepared herein is no more than 2%, preferably no more than 1%, more preferably no more than 0.5%, even more preferably no more than 0.1% by weight of the alkoxylate composition.

As used herein the term "reducing the level of 1,4-dioxane" means that the level of 1,4-dioxane present in the alkoxylate composition prepared using a DMC catalyst is no more than 10%, preferably no more than 1%, and even more preferably no more than 0.1% of the level of 1,4-dioxane present in an equivalent alkoxylate composition prepared using a Lewis acid alkoxylation catalyst. Typically, this means that the amount of 1,4-dioxane in the alkoxylate composition prepared herein is no more than 100 ppm (wt/wt), preferably no more than 10 ppm (wt/wt), more preferably no more than 5 ppm (wt/wt).

As used herein the term "equivalent alkoxylate composition" means an alkoxylate composition which comprises an alkoxylate compound having substantially the same chemical composition (i.e. same average carbon chain length, same average degree of ethoxylation, and the like) as the alkoxylate compound present in the composition prepared according to the present invention, and wherein the alkoxylate compositions are present in the same amount by weight.

The alkoxylate composition herein is prepared by reacting a starting alcohol with an alkylene oxide in the presence of a DMC catalyst.

The alcohols suitably utilized in the process of the present invention include those known in the art for reaction with alkylene oxides and conversion to alkoxylate products.

Suitable starting alcohols for use in the preparation of an alkoxylated alcohol composition herein include those known in the art for reaction with alkylene oxides and conversion to alkoxylated alcohol products, including both mono- and poly-hydroxy alcohols.

Acyclic aliphatic mono-hydric alcohols (alkanols) form a most preferred class of reactants, particularly the primary alkanols, although secondary and tertiary alkanols are also very suitably utilized in the preparation of the alkoxylated alcohol composition herein. As is often the case for alkoxylation reactions, primary alcohols are more reactive, and in some cases substantially more reactive than the corresponding secondary and tertiary compounds. However, it has surprisingly been found by the present inventors that the DMC catalysts used herein are particularly suitable for the direct ethoxylation of secondary alcohols as well as primary alcohols. It is particularly useful to be able to directly ethoxylate secondary alcohols since secondary alcohols can be derived from relatively cheap feedstocks such as paraffins (by oxidation) or from short chain C₆-C₁₀ primary alcohols (by propoxylation). Suitable paraffins for producing secondary alcohols are, for example, those produced from Fischer-Tropsch technologies.

Preference can also be expressed, for reasons of both process performance and commercial value of the product, for alkanols having from 9 to 30 carbon atoms, with C₉ to C₂₄ alkanols considered more preferred and C₉ to C₂₀ alkanols considered most preferred, including mixtures thereof, such as a mixture of C₉ and C₂₀ alkanols. As a general rule, the alkanols may be of branched or straight chain structure depending on the intended use. In one embodiment, preference further exists for alkanol reactants in which greater than 50 percent, more preferably greater than 60 percent and most preferably greater than 70 percent of the molecules are of linear (straight chain) carbon structure. In another embodiment, preference further exists for alkanol reactants in which greater than 50 percent, more preferably greater than 60 percent and most preferably greater than 70 percent of the molecules are of branched carbon structure.

The general suitability of such alkanols as reactants in alkoxylation reactions is well recognized in the art. Commercially available mixtures of primary monohydric alkanols prepared via the oligomerisation of ethylene and the hydroformylation or oxidation and hydrolysis of the resulting higher olefins are particularly preferred. Examples of commercially available alkanol mixtures include the NEODOL Alcohols, trademark of and sold by Shell Chemical Company, including mixtures of C₉, C₁₀ and C₁₁ alkanols (NEODOL 91 Alcohol), mixtures of C₁₂ and C₁₃ alkanols (NEODOL 23 Alcohol), mixtures of C₁₂, C₁₃, C₁₄ and C₁₅ alkanols (NEODOL 25 Alcohol), and mixtures of C₁₄ and C₁₅ alkanols (NEODOL 45 Alcohol, and NEODOL 45E Alcohol); the ALFOL Alcohols (ex. Vista Chemical Company), including mixtures of C₁₀ and C₁₂ alkanols (ALFOL 1012), mixtures of C₁₂ and C₁₄ alkanols (ALFOL 1214), mixtures of C₁₆ and C₁₈ alkanols (ALFOL 1618), and mixtures of C₁₆, C₁₈ and C₂₀ alkanols (ALFOL 1620), the EPAL Alcohols (Ethyl Chemical Company), including mixtures of C₁₀ and C₁₂ alkanols (EPAL 1012), mixtures of C₁₂ and C₁₄ alkanols (EPAL 1214), and mixtures of C₁₄, C₁₆ and C₁₈ alkanols (EPAL 1418), and the TERGITOL-L Alcohols (Union Carbide), including mixtures of C₁₂, C₁₃, C₁₄ and C₁₅ alkanols (TERGITOL-L 125). Also suitable for use herein is NEODOL 1, which is primarily a C₁₁ alkanol. Also very suitable are the commercially available alkanols prepared by the reduction of naturally occurring fatty esters, for example, the CO and TA products of Proctor and Gamble Company and the TA alcohols of Ashland Oil Company.

As mentioned above, secondary alcohols are also a preferred class of reactants for use herein. Examples of secondary alcohols suitable for use herein include 2-undecanol, 2-hexanol, 3-hexanol, 2-heptanol, 3-heptanol, 2-octanol, 3-octanol, 2-nonanol, 2-decanol, 4-decanol, 2-dodecanol, 2-tetradecanol, 2-hexadecanol, and mixtures thereof.

Mixtures of alcohols comprising primary and secondary alcohols are also suitable for use herein.

In particular, oxidation products arising from Fischer-Tropsch derived paraffins (which may include mixtures of primary and secondary alcohols) are particularly suitable for use herein.

Suitable alkylene oxide reactants for use herein include an alkylene oxide (epoxide) reactant which comprises one or more vicinal alkylene oxides, particularly the lower alkylene oxides and more particularly those in the C₂ to C₄ range. In general, the alkylene oxides are represented by the formula (VII) 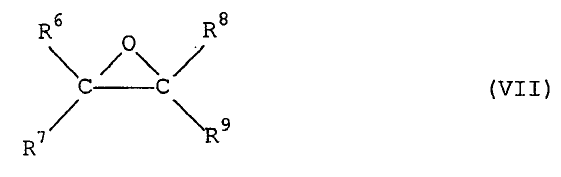 wherein each of the R⁶, R⁷, R⁸ and R⁹ moieties is individually selected from the group consisting of hydrogen and alkyl moieties. Reactants which comprise ethylene oxide, propylene oxide, butylene oxide, or mixtures thereof are more preferred, particularly those which consist essentially of ethylene oxide and propylene oxide. Alkylene oxide reactants consisting essentially of ethylene oxide are considered most preferred from the standpoint of commercial opportunities for the practice of alkoxylation processes, and also from the standpoint of the preparation of products having narrow-range ethylene oxide adduct distributions.

For preparation of the alkoxylate compositions herein the alkylene oxide reactant and the starting active hydrogen containing organic compound are contacted in the presence of a suitable DMC catalyst. The catalyst is applied in an amount which is effective to catalyse the alkoxylation reaction. Preferably the catalyst is used at a level such that the level of solid DMC catalyst remaining in the final alkoxylate composition is in the range from 1 to 1000 ppm (wt/wt), preferably of from 5 to 200 ppm (wt/wt), more preferably from 10 to 100 ppm (wt/wt).

The catalyst used for the preparation of the alkoxylate composition of the present invention is a double metal cyanide catalyst. Any double metal cyanide catalyst suitable for use in alkoxylation reactions can be used in the present invention.

Conventional DMC catalysts are prepared by reacting aqueous solutions of metal salts and metal cyanide salts or metal cyanide complex acids to form a precipitate of the DMC compound.

Suitable metal salts and metal cyanide salts are, for instance, described in EP-A-755716. Thus, suitable metal salts are water-soluble salts suitably having the formula M(X')_{n'}, in which M is selected from the group consisting of Zn(II), Fe(II), Ni(II), Mn(II), Co(II), Sn(II), Pb(II), Fe(III), Mo(IV), Mo(VI), Al(III), V(V), V(IV), Sr(II), W(IV), W(VI), Cu(II), and Cr(III). More preferably, M is selected from the group consisting of Zn(II), Fe(II), Co(II), and Ni(II), especially Zn(II). In the formula, X' is preferably an anion selected from the group consisting of halide, hydroxide, sulfate, carbonate, cyanide, oxalate, thiocyanate, isocyanate, isothiocyanate, carboxylate, and nitrate. The value of n' satisfies the valency state of M and typically is from 1 to 3. Examples of suitable metal salts include, but are not limited to, zinc chloride, zinc bromide, zinc acetate, zinc acetonylacetate, zinc benzoate, zinc nitrate, iron(II) chloride, iron(II) sulfate, iron(II) bromide, cobalt(II) chloride, cobalt(II) thiocyanate, nickel(II) formate, nickel(II) nitrate, and the like, and mixtures thereof. Zinc halides, and particularly zinc chloride, are preferred.

The metal cyanide salt is a water-soluble metal cyanide salt preferably having the general formula (Y)ₐ'M'(CN)_{b'} (A')_{c'} in which M' is selected from the group consisting of Fe(II), Fe(III), Co(II), Co(III), Cr(II), Cr(III), Mn(II), Mn(III), Ir(III), Ni(II), Rh(III), Ru(II), V(IV), and V(V). More preferably, M' is selected from the group consisting of Co(II), Co(III), Fe(II), Fe(III), Cr(III), Ir(III), and Ni(II), especially Co(II) or Co(III). The water-soluble metal cyanide salt can contain one or more of these metals. In the formula, Y is an alkali metal ion or alkaline earth metal ion, such as lithium, sodium, potassium and calcium. A' is an anion selected from the group consisting of halide, hydroxide, sulfate, carbonate, cyanide, oxalate, thiocyanate, isocyanate, isothiocyanate, carboxylate, and nitrate. Both a' and b' are integers greater than or equal to 1; c' can be 0 or an integer; the sum of the charges of a', b', and c' balances the charge of M'. Suitable water-soluble metal cyanide salts include, for instance, potassium hexacyanocobaltate(III), potassium hexacyanoferrate(II), potassium hexacyanoferrate(III), calcium hexacyanocobaltate(III) and lithium hexacyanoiridate(III). A particularly preferred water-soluble metal cyanide salt for use herein is potassium hexacyanocobaltate(III).

DMC catalyts are usually prepared in the presence of a low molecular weight organic complexing agent such that a dispersion is formed comprising a solid DMC complex in an aqueous medium. The organic complexing agent used should generally be reasonably to well soluble in water. Suitable complexing agents are, for instance, disclosed in EP-A-555053 and in general are water-soluble heteroatom-containing organic compounds that can complex with the double metal cyanide compound. Thus, suitable complexing agents include alcohols, aldehydes, ketones, ethers, esters, amides, ureas, nitriles, sulfides, and mixtures thereof. Preferred complexing agents are ethers like dimethoxyethane and diglyme and water-soluble aliphatic alcohols, such as ethanol, isopropyl alcohol, n-butyl alcohol (1-butanol), isobutyl alcohol (2-methyl-1-propanol), sec-butyl alcohol (2-butanol), and tert-butyl alcohol (2-methyl-2-propanol). Of these, dimethoxyethane and tert-butyl alcohol are most preferred, especially tert-butyl alcohol.

Combining both aqueous reactant streams may be conducted by conventional mixing techniques including mechanical stirring and ultrasonic mixing. Although applicable, it is not required that intimate mixing techniques like high shear stirring or homogenization are used. The reaction between metal salt and metal cyanide salt may be carried out at a pressure of from 0.5 to 10 bar and a temperature of from 0 to 80 °C. However, it is preferred that the reaction be carried out at mild conditions, i.e. a pressure of 0.5 to 2 bar and a temperature of from 10 to 40°C.

After the reaction has taken place and a DMC compound has been formed it is preferable to add an extracting liquid to the dispersion of solid DMC complex in aqueous medium, in order that the DMC catalyst particles can be efficiently and easily separated from the aqueous phase without losing any catalytic activity.

A preferred process for preparing the DMC catalyst herein comprises the steps of
(a) combining an aqueous solution of a metal salt with an aqueous solution of a metal cyanide salt and reacting these solutions, wherein at least part of this reaction takes place in the presence of an organic complexing agent, thereby forming a dispersion of a solid DMC complex in an aqueous medium;
(b) combining the dispersion obtained in step (a) with an extracting liquid, which is essentially insoluble in water and which is capable of extracting the solid DMC complex formed in step (a) from the aqueous medium, and allowing a two-phase system to be formed consisting of a first aqueous layer and a layer containing the DMC complex and the liquid added;
(c) removing the first aqueous layer; and
(d) recovering the DMC catalyst from the layer containing the DMC catalyst.

Suitable extracting liquids are described in WO01/72418. A suitable extracting liquid should meet two requirements: firstly it should be essentially insoluble in water and secondly it must be capable of extracting the DMC complex from the aqueous phase. The latter requirement implies that the organic complexing agent used must have a preference for interacting with this extracting liquid over the aqueous phase containing the dissolved salts. Namely, it is believed that the complexing agent interacts with the extracting liquid and in fact drags along the DMC complex from the aqueous phase into the phase formed by the extracting liquid. The extracting liquid can, for instance, be an ester, a ketone, an ether, a diester, an alcohol, a di-alcohol, a (di)alkyl carbamate, a nitrile or an alkane.

Preferably, the extracting liquid used comprises a compound of the general formula (VI): 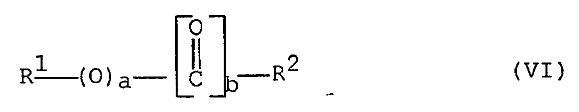 wherein:
R¹ represents hydrogen, an aryl group, a substituted or unsubstituted C₁-C₁₀ alkyl group or a group R³-NH-,
R² represents hydrogen, an optionally halogenated C₁-C₁₀ alkyl group, a group R³-NH-, a group -R⁴-C(O)O-R⁵ or a cyanide group,
R³ represents hydrogen or a C₁-C₁₀ alkyl group,
R⁴ represents a substituted or unsubstituted alkylene group having 2 to 15 carbon atoms,
R⁵ represents hydrogen, a substituted or unsubstituted C₁-C₁₀ alkyl group, and
a and b independently are 0 or 1.

In a first preferred embodiment in the general formula (VI) R¹ represents hydrogen, a=1, b=0 and R² represents a group -R⁴-OH with R⁴ representing an alkylene group having 3 to 10 carbon atoms. A specific example of this preferred compound includes 2-butyl-2-ethyl-1,3-propanediol.

In a second preferred embodiment of the present invention in the general formula (VI) R¹ and R² independently represent an alkyl group having 1 to 5 carbon atoms, a=1 and b=0. Preferred examples of this embodiment are diethyl ether, methyl tert-butyl ether, di-isopropyl ether and dibutyl ether. Of these methyl tert-butyl ether is particularly preferred.

In a third preferred embodiment in the general formula (VI) R¹ represents an alkyl group having 1 to 6 carbon atoms, a=1, b=1 and R² represents hydrogen or an alkyl group having 1 to 6 carbon atoms or a group -R⁴-C(O)O-R⁵ with R⁴ being a substituted or unsubstituted alkylene group having 3 to 15 carbon atoms and R⁵ being an alkyl group having 1 to 5 carbon atoms. The group R⁴ may contain alicyclic, aliphatic (alkyl) or polar substituents, like C₁-C₄ alkoxy groups. Suitably R⁴ is a 1,3-propylene group with one or two substituents on the middle carbon atom. Preferred examples of this embodiment are ethyl formiate, ethyl acetate, ethyl-2-ethyl-3-methyl butanoate, di-ethyl malonate and di-ethyl-2-cyclohexyl-2-propyl malonate.

In a fourth preferred embodiment in the general formula (VI) R¹ and R² independently represent an alkyl group having 1 to 5 carbon atoms, a=0 and b=0. Thus, in this embodiment the extracting liquid is an alkane having from 2 to 10 carbon atoms. Heptane was found to be particularly useful for the purpose of the present invention.

In a fifth preferred embodiment in the general formula (VI) R¹ represents an aryl group, suitably a phenyl group, or an alkyl group having 1 to 5 carbon atoms, R² represents a cyanide group, a=0 and b=0. Preferred examples of this embodiment are benzonitrile and pivalonitrile (tert-butylnitrile).

In a sixth preferred embodiment R¹ and R² independently represent a group R³-NH- with R³ being hydrogen or a C₁-C₁₀ alkyl group, a=0 and b=1. Preferred examples of this embodiment are butyl carbamate, dibutyl carbamate and propyl carbamate.

In a seventh preferred embodiment R¹ represents hydrogen, R² represents a halogen-substituted C1-C5 alkyl group, a=0 and b=0. Preferred examples of this embodiment are dichloromethane, 1,2-dichloroethane and tetrachloroethane.

An especially preferred extracting liquid for use herein is methyl tert-butyl ether.

Typically the extracting liquid is added under stirring and stirring is continued until the liquid has been uniformly distributed through the reaction mixture. Stirring time is not critical and may suitably take from 10 seconds up to 2 hours. It is considered beneficial from a process economic view to keep the stirring time as short as possible and therefore, stirring time will typically be from 30 seconds to 30 minutes.

After the stirring has stopped the reaction mixture is allowed sufficient time to settle, i.e. sufficient time to separate into two phases: an aqueous bottom layer and a layer floating thereon containing the DMC catalyst dispersed in the extracting liquid.

The amount of the extracting liquid added should be sufficient to effect phase separation. Accordingly, normally at least 1% by weight, preferably at least 2% by weight and more preferably at least 3% by weight based on total weight of the alkoxylate reaction product of extracting liquid are added. Any amount of extracting liquid above the minimum amount required to effect phase separation can be used. The maximum amount will usually be determined by hardware constraints like volume of the reactor. Typically, however, the amount of extracting solvent added will not exceed 50% by weight, suitably 30% by weight and more suitably 20% by weight based on total weight of the alkoxylate reaction product. The addition is suitably carried out at a temperature of from 0 to 80 °C, suitably 10 to 50 °C. The pressure may be the same as during the alkoxylation reaction.

The next part of the catalyst preparation process is for the aqueous layer to be removed. Since the aqueous layer forms the bottom layer of the two phase system formed, this could e.g. be easily accomplished by draining the aqueous layer via a valve in the bottom part of the vessel in which the phase separation occurred. In addition to water the aqueous layer will typically contain the excess complexing agent used (i.e. that amount of complexing agent which is not attached to the DMC complex), the water-soluble salts like the unreacted metal salt (e.g. zinc chloride) and any water-soluble salt formed during the reaction between metal salt and metal cyanide salt (e.g. potassium chloride and cobalt salts) and possibly a small amount of the extracting compound left in the aqueous phase. Normally the aqueous layer removed will constitute from 10 to 90 volume% of the total volume of liquid plus catalyst particles present in the vessel, but the volume ratio of aqueous layer to extracting compound layer is not critical for the workability of the present invention. The exact ratio will normally be determined by hardware constraints. After removal of the aqueous phase the remaining phase contains the solid DMC catalyst particles, which are dispersed or finely divided in the extracting compound and which are subsequently recovered.

The catalyst recovery step can be carried out in various ways. Such recovery procedure will normally involve mixing the DMC catalyst with complexing agent, optionally in admixture with water, and separating DMC catalyst and complexing agent/water again, e.g. by filtration, centrifugation/decantation or flashing. This procedure may be repeated one or more times. Eventually, the catalyst is dried and recovered as a solid. As disclosed in WO-A-97/40086 and EP-A-700949, the final solid catalyst can also be recovered as a composition also containing 5 to 80% by weight of polyether having a molecular weight of respectively less than 500 and greater than 500. The recovery step suitably comprises adding a water/complexing agent to the DMC catalyst layer and admixing catalyst layer and water/complexing agent (e.g. by stirring), allowing a two phase system to be formed and removing the aqueous layer. This procedure may be repeated one to five times after which the remaining catalyst layer may be dried and the catalyst may be recovered in solid form (as a powder) or, alternatively, a liquid alcohol may be added to the catalyst layer and a catalyst suspension in liquid alcohol is formed, which can be used as such.

In one embodiment a preferred catalyst recovery step comprises the steps of
(1) admixing organic complexing agent, water and optionally additional extracting liquid with the layer containing the DMC catalyst and allowing a two phase system to be formed consisting of a second aqueous layer and a layer containing the DMC catalyst;
(2) removing the second aqueous layer;
(3) optionally repeating steps (1) and (2) one to five times, suitably one or two times;
(4) adding organic complexing agent to layer containing the DMC catalyst while stirring; and
(5) removing the complexing agent (e.g. by flashing or stripping) and recovering the DMC catalyst as solid particles.
   In another embodiment the recovery step comprises steps (1) to (4) as defined above followed by:
(5') adding a liquid alcohol or liquid polyol to the product of step (4), thereby forming a slurry of DMC catalyst in a liquid medium of alcohol/organic complexing agent;
(6) removing the organic complexing agent; and
(7) recovering the DMC catalyst as a suspension in the liquid alcohol or liquid polyol.

The amount of water used in step (1) of the catalyst recovery step should be sufficient to form an aqueous layer. The organic complexing agent and water and optionally additional extracting liquid may be added as separate streams or as a mixture in a single stream. Additional extracting liquid may be added to compensate for any small amount left in the aqueous phase. If added, it will be in small amounts. The weight ratio of complexing agent to water suitably ranges from 5:95 to 50:50, more suitably from 10:90 to 40:60. The total amount of water and complexing agent added is not critical and could, for instance, correspond with up to 20 volume% more or less than the amount of aqueous layer drained in step (3). Water and complexing agent are effectively admixed with the DMC catalyst layer, for instance by mechanical stirring. After effective mixing has taken place the resulting mixture is allowed to settle, so that a two phase system can be formed. Once this has happened the aqueous (bottom) layer is removed in step (2) of the catalyst recovery stage. The procedure may be repeated one to five times, suitably one or two times.

In step (4) of the recovery stage pure organic complexing agent is added to the DMC catalyst layer while stirring in an amount which corresponds with the amount of aqueous layer drained in the preceding step, although 20 volume% more or less would still be acceptable.

In subsequent step (5) of the recovery stage the complexing agent may be removed by stripping or flashing, thus recovering the DMC catalyst as a solid. The complexing agent may, for instance, be flashed off at atmospheric conditions or under reduced pressure. Flashing under reduced pressure is preferred, because this enables separation at a lower temperature which reduces the risk of thermal decomposition of the DMC catalyst. In a particularly preferred embodiment the organic complexing agent is removed by flashing under vacuum at a temperature of 50 to 80 °C. Together with the complexing agent traces of water and extracting liquid, which were still present in the mixture are also removed. The DMC catalyst is recovered as a solid and may be subjected to a subsequent drying treatment.

Alternatively, step (5') of the recovery stage comprises adding an alcohol or polyol in an amount sufficient to form a catalyst slurry of DMC catalyst in a liquid medium of alcohol/polyol and complexing agent. Suitably, the amount of alcohol or polyol is such that the solids content of slurry formed is from 0.01 to 50% by weight, more suitably from 1 to 50% by weight, even more suitably 1 to 30% by weight and most suitably 1 to 10% by weight.

The alcohol/polyol added may be any liquid alcohol/polyol which is suitable to serve as a liquid medium for the DMC catalyst particles. When the DMC catalyst is used for catalysing the alkoxylation reaction of alcohols, it is preferred to use an alcohol which is compatible with the alkoxylated alcohols to be produced and which will not have any negative effect on the final alkoxylated alcohol produced when present therein in trace amounts. Therefore, it is particularly preferred to use an alcohol similar to the alkoxylated alcohol to be produced by the DMC catalyst. Examples of suitable alcohols include the NEODOL (RTM) alcohols commercially available from the Shell Chemical Company, however, it is noted that for NEODOL 45 (RTM) higher than ambient temperatures are required as it has a melting point of 29 - 32 °C.

Examples of suitable polyols include polyols such as polyethylene glycol and polypropylene glycol.

In the subsequent step (6) of the recovery stage the organic complexing agent is removed from the catalyst slurry. This can be achieved by any means known in the art to be suitable for liquid-liquid separation. A preferred method for the purpose of the present invention is flashing off the complexing agent at atmospheric conditions or under reduced pressure. Flashing under reduced pressure is preferred, because this enables separation at a lower temperature which reduces the risk of thermal decomposition of the DMC catalyst. In a particularly preferred embodiment the organic complexing agent is removed by flashing under vacuum at a temperature of 50 to 80 °C. Together with the complexing agent traces of water and extracting liquid, which were still present in the mixture are also removed.

Finally, in step (7) of the recovery stage the DMC catalyst is recovered as a slurry in liquid alcohol/polyol. The advantage of such a slurry is that it is storage stable and can, for instance, be stored in a drum. Moreover, dosing of the catalyst and its distribution through the alkoxylation medium is greatly facilitated by using a catalyst slurry.

When using zinc chloride as the metal salt and potassium hexacyanocobaltate as the metal cyanide salt, such catalysts will typically have the formula (V):

Zn₂[Co(CN)₆]Cl.nC.mH₂O.pA (V)

wherein C is the complexing agent used and A is the compound of general formula (VI) used. Preferably, C is tert-butyl alcohol and A is methyl tert-butyl ether, di-ethyl ether, di-isopropyl ether, tert-amyl methyl ether or di-butyl ether, preferably methyl tert-butyl ether. Preferably, n is of from 0 to 10, m is of from 0 to 20 and p is of from 0 to 10.

As mentioned above, the use of a DMC catalyst for preparing an alkoxylate composition provides a composition having reduced levels of free alcohol and reduced levels of 1,4-dioxane, compared to conventionally prepared alkoxylate compositions (e.g. those prepared using a basic KOH catalyst and an acid catalyst respectively).

Hence, according to a further embodiment of the present invention the alkoxylate composition comprises:
an alkoxylate compound having the empirical formula (IV):

   R'X(EO)ₓ(PO)_{y}(BO)_{z}H (IV)
wherein R' is an alkyl group having from 8 to 30 carbon atoms, X represents an oxygen atom, EO represents an ethyleneoxy moiety, PO represents a propyleneoxy moiety, BO represents a butyleneoxy moiety, x is in the range of from 0 to 70, preferably from 1 to 30, even more preferably from 1 to 15, y is in the range of from 0 to 70 preferably from 1 to 30, even more preferably from 1 to 15 and z is in the range of from 0 to 70 preferably from 1 to 30, even more preferably from 1 to 15, and the sum of x, y and z is in the range of from 1 to 70, preferably from 1 to 30, even more preferably from 1 to 15;
wherein the alkoxylate composition is prepared by the reaction of an alcohol having a formula R'-XH wherein R' and X are as defined above, with an alkylene oxide selected from ethylene oxide, propylene oxide, butylene oxide and mixtures thereof, in the presence of a double metal cyanide complex catalyst, and wherein the alkoxylate composition comprises no more than 2% by weight of the alkoxylate product composition, of starting alcohol R'-XH and no more than 100 ppm (wt/wt) 1,4-dioxane compound.

In preferred alkoxylate compositions herein, the amount of free alcohol is no more than 1%, more preferably no more than 0.5%, even more preferably no more than 0.1% by weight of the alkoxylate composition, and the amount of 1,4-dioxane in the alkoxylate composition is no more than 10 ppm (wt/wt), more preferably no more than 5 ppm (wt/wt).

In a particularly preferred embodiment herein, the active hydrogen containing organic compound is a primary alcohol. In another particularly preferred embodiment herein, the active hydrogen containing organic compound is a secondary alcohol.

In a further preferred embodiment, the alkoxylate composition produced herein comprises an alkoxylated alcohol having the empirical formula (VIII):

R'O(EO)ₓ(PO)_{y}(BO)_{z}H (VIII)

wherein R' is an alkyl group having from 8 to 30 carbon atoms, EO represents an ethyleneoxy moiety, PO represents a propyleneoxy moiety, BO represents a butyleneoxy moiety, x is in the range of from 0 to 70, preferably from 1 to 30, even more preferably from 1 to 15, y is in the range of from 0 to 70 preferably from 1 to 30, even more preferably from 1 to 15 and z is in the range of from 0 to 70 preferably from 1 to 30, even more preferably from 1 to 15, and the sum of x, y and z is in the range of from 1 to 70, preferably from 1 to 30, even more preferably from 1 to 15.

In a preferred embodiment, x is in the range of from 1 to 70, preferably from 1 to 30, even more preferably from 1 to 15, y is 0 and z is 0 (i.e. an ethoxylated alcohol).

In a further preferred embodiment R' is a primary alkyl group, i.e. the -O- is attached to the R' group via a primary carbon atom. In another preferred embodiment, R' is a secondary alkyl group, i.e. the -O- is attached to the R' group via a secondary carbon atom.

In a final aspect the present invention also relates to a process for the preparation of an alkoxylate composition comprising reacting a starting alcohol having the formula R'-XH wherein R' and X are as defined above with an alkylene oxide selected from ethylene oxide, propylene oxide, butylene oxide and mixtures thereof in the presence of a DMC catalyst wherein the double metal cyanide catalyst is prepared by a process comprising the steps of
(a) combining an aqueous solution of a metal salt with an aqueous solution of a metal cyanide salt and reacting these solutions, wherein at least part of this reaction takes place in the presence of an organic complexing agent, thereby forming a dispersion of a solid DMC complex in an aqueous medium;
(b) combining the dispersion obtained in step (a) with an extracting liquid, which is essentially insoluble in water and which is capable of extracting the solid DMC complex formed in step (a) from the aqueous medium, and allowing a two-phase system to be formed consisting of a first aqueous layer and a layer containing the DMC complex and the liquid added;
(c) removing the first aqueous layer; and
(d) recovering the DMC catalyst from the layer containing the DMC catalyst.

In one preferred process for the preparation of the alkoxylate composition R'XH is a primary alcohol. In another preferred process for the preparation of the alkoxylate composition R'XH is a secondary alcohol.

The DMC catalysts used in the present invention are very active and hence exhibit high alkoxylation rates. They are sufficiently active to allow their use at very low concentrations, preferably of from 5 to 200 ppm (wt/wt), more preferably from 10 to 100 ppm (wt/wt) of the solid catalyst content in the final alkoxylation product composition. At such low concentrations, the catalyst can often be left in the alkoxylated alcohol composition without an adverse effect on product quality. The ability to leave catalysts in the alkoxylated alcohol composition is an important advantage because commercial alcohols currently require a catalyst removal step. The concentration of the residual cobalt in the final alkoxylate composition is preferably below 10 ppm (wt/wt).

The invention will be further illustrated by the following examples, however, without limiting the invention to these specific embodiments.

### Examples

### Example 1

### Preparation of a DMC catalyst slurried in polypropylene glycol 400

30 g of zinc chloride (220 mmol zinc chloride, containing 0.2 mmol zinc oxide) was dissolved in 100 g of de-ionised water at room temperature. The resulting solution was charged to a 1 litre glass reactor, equipped with a triple pitched bladed turbine. Subsequently, 117 g of tert-butyl alcohol (TBA) and 95 g of de-ionised water were added.

A second solution of 12 g potassium hexacyanocobaltate dissolved in 225 g of de-ionised water, was added, over the course of 30 minutes, to the zinc chloride solution. The reactor contents were well stirred during the addition. After the addition, the reactor contents were stirred for a subsequent 30 minutes and then allowed to stand overnight.

The following day, tert-butyl methyl ether (MTBE) (12 %wt. on reactor contents) was added and the reactor contents were stirred for 5 minutes. The reactor contents were then allowed to stand for 30 minutes, after which time, the lower (aqueous) liquid layer of the reactor contents (337 g) were removed.

To the reactor, a 25/75 wt./wt. mixture of TBA and de-ionised water was introduced (337 g). The reactor contents were then stirred for 5 minutes. The reactor contents were then allowed to stand for 30 minutes, after which time, the lower (aqueous) liquid layer of the reactor contents (355 g) were removed.

To the reactor, a 25/75 wt./wt. mixture of TBA and de-ionised water (355 g), with an additional MTBE (2.5 %wt. on initial reactor contents) was introduced. The reactor contents were then stirred for 5 minutes. The reactor contents were then allowed to stand for 30 minutes, after which time, the lower (aqueous) liquid layer of the reactor contents (308 g) were removed.

308 g of TBA was subsequently introduced and the reactor contents were stirred for 30 minutes.

The solids content of the resulting suspension was measured. The measurement was performed by weighing a sample of the suspension and subsequently removing volatile components (MTBE, TBA and water) by stripping, under vacuum and a nitrogen purge at 50 °C, until a constant weight is obtained. Based on the solids content, polypropylene glycol 400 (PPG-400) was added to obtain a 3 %wt. solution of zinc hexacyanocobaltate complex in PPG-400.

The reactor contents were subsequently stirred for 30 minutes, after which time, the MTBE, TBA and water were removed, by stripping at 80 °C and 50 mbar.

The resulting product had a water content of less than 0.5 %wt.

### Example 2

### Preparation of a DMC catalyst slurried in NEODOL 1

29.3 g of technical grade zinc chloride (220 mmol zinc chloride, containing 2.2 mmol zinc oxide) and 8.8 mmol of zinc oxide were dissolved in 100 g of de-ionised water at room temperature. The resulting solution was charged to a 1 litre glass reactor, equipped with a triple pitched bladed turbine. Subsequently, 117 g of TBA and 95 g of de-ionised water were added.

A second solution of 12 g potassium hexacyanocobaltate dissolved in 225 g of de-ionised water, was added, over the course of 30 minutes, to the zinc chloride solution. The reactor contents were well stirred during the addition. After the addition the reactor contents were stirred for a subsequent 30 minutes and then allowed to stand overnight.

The following day, MTBE (12 %wt. on reactor contents) was added and the reactor contents were stirred for 5 minutes. The reactor contents were then allowed to stand for 30 minutes, after which time, the lower (aqueous) liquid layer of the reactor contents (369 g) were removed.

To the reactor, a 25/75 wt./wt. mixture of TBA and de-ionised water was introduced (369 g). The reactor contents were then stirred for 5 minutes. The reactor contents were then allowed to stand for 30 minutes, after which time, the lower (aqueous) liquid layer of the reactor contents (307 g) was removed.

To the reactor, a 25/75 wt./wt. mixture of TBA and de-ionised water (307 g), with an additional MTBE (2.5 %wt. on initial reactor contents) was introduced. The reactor contents were then stirred for 5 minutes. The reactor contents were then allowed to stand for 30 minutes, after which time, the lower (aqueous) liquid layer of the reactor contents (268 g) were removed.

268 g of TBA was subsequently introduced and the reactor contents were stirred for 30 minutes.

The solids content of the resulting suspension was measured. The measurement was performed by weighing a sample of the suspension and subsequently removing volatile components (MTBE, TBA and water) by stripping, under vacuum and a nitrogen purge at 50 °C, until a constant weight is obtained. Based on the solids content, NEODOL 1 (RTM), a C₁₁ primary alcohol composition commercially available from The Shell Chemical Company, was added to obtain a 3 %wt. solution of zinc hexacyanocobaltate complex in NEODOL 1.

The reactor contents were subsequently stirred for 30 minutes, after which time, the MTBE, TBA and water were removed, by stripping at 60 °C and 50 mbar.

The resulting product had a water content of less than 0.5 %wt.

### Example 3

### Preparation of an ethoxylated C11 alcohol having an average of 5 ethyleneoxy groups per molecule

A 5 litre stirred tank reactor was charged with 1667 g of NEODOL 1 (a C11 primary alcohol composition commercially available from the Shell Chemical Company) and 6.67 g of the catalyst slurry in NEODOL 1, formed by the process described in Example 2. Under constant stirring, the reactor tank was flushed three times with nitrogen, by raising the pressure within the reactor tank to 5 bara and subsequently releasing the pressure to atmospheric pressure. The reactor contents were heated, under a nitrogen atmosphere, to a temperature of 130 °C, and the pressure of nitrogen was increased to 2.6 bara.

2132 g of ethylene oxide (EO) was introduced to the reactor contents such that the addition of the first 10 % of the EO, the pressure within the tank reactor did not exceed 4.6 bara. The remaining 90 % was subsequently added linearly over a 2 hour period, during which time the pressure increased due to the compression of the nitrogen gascap in the reactor.

After all of the EO had been introduced into the reactor contents, the reactor contents were held at the reaction temperature for half an hour, allowing the reaction of residual EO. The reactor contents were allowed to cool to a temperature of 100 °C.
Subsequently, any residual EO was stripped off with nitrogen at 30 mbara for 15 minutes.
The total amount of product composition formed was 3800 g.

Measurement of the average number of moles of EO per mole of NEODOL 1 and residual free alcohol was performed using high performance liquid chromatography (HPLC). The technique for these measurements involved derivatising the ethoxylated alcohol using 4-nitrobenzoylchloride. The product is then analysed by Gradient Elution High Performance Liquid Chromatography using a Polygosil Amino stationary phase with an isohexane/ethylacetate/acetonitrile mobile phase. Detection was performed by ultra-violet absorbance. Quantification is by means of an internal normalisation technique.

The concentration of 1,4-dioxane in the final product composition was measured by Gas Chromatography (GC). The technique for these GC measurements involves introducing a known amount of the alcohol ethoxylate product into a sealed vial, which is thermostated and held for 20 minutes at 50 °C to allow equilibrium between the gas and liquid phases. After thermostating is complete, the product composition vapour is automatically injected into the Capillary Gas Chromatography aparatus. As the analytical column, a fused silica, 50 m × 0.32 mm internal diameter, 1.0 µm film CpSil 5CB is used. Helium is employed as the carrier gas. Detection was performed by flame ionization. The calibration is performed using the standard addition method at 2 levels. The results are shown in Table 1 below.

The intake of catalyst (solids) on total intake was 50 ppm (wt/wt), giving rise to a concentration of cobalt remaining in the product composition of 5.5 ppm (wt/wt), and a concentration of zinc remaining in the product composition of 12.7 ppm (wt/wt), as measured by Inductively Coupled Plasma - Mass Spectroscopy (ICP-MS).

### Example 4

### Preparation of an ethoxylated C11 alcohol having an average of 7 ethyleneoxy groups per molecule

A 5 litre stirred tank reactor was charged with 1369 g of NEODOL-1 (a C11 primary alcohol composition commercially available from the Shell Chemical Company) and 6.33 g of the catalyst slurry in NEODOL-1, formed by the process described in Example 2. Under constant stirring, the reactor tank was flushed three times with nitrogen, by raising the pressure within the reactor tank to 5 bara and subsequently releasing the pressure to atmospheric pressure. The reactor contents were heated, under a nitrogen atmosphere, to a temperature of 130 °C, and the pressure of nitrogen was increased to 2.2 bara.

2438 g of EO was introduced to the reactor such that during the addition of the first 10 % of the EO, the pressure within the tank reactor did not exceed 3.9 bara. The remaining 90 % was subsequently added linearly over a 2 hour period, during which time the pressure increased due to the compression of the nitrogen gascap in the reactor.

After all of the EO had been introduced to the reactor contents, the reactor contents were held at the reaction temperature for a further 1 hour, allowing the reaction of residual EO. The reactor contents were allowed to cool to a temperature of 100 °C.
Subsequently, any residual EO was stripped off with nitrogen at 30 mbara for 15 minutes.

The total amount of product composition formed was 3800 g. The average number of moles of EO per molecule, level of free alcohol and level of 1,4-dioxane were measured using the same methods as used in Example 3. The results are shown in Table 1 below.

The catalyst (solids) intake on total intake of the reactor was 50 ppm (wt/wt), leading to a concentration of cobalt remaining in the product composition of 4.5 ppm (wt/wt), and the concentration of zinc remaining in the product composition of 10.6 ppm (wt/wt).

### Example 5

### Preparation of an ethoxylated C14/C15 alcohol with an average of 7 ethyleneoxy groups per molecule)

A 1.25 litre stirred tank reactor was charged with 241.3 g of NEODOL 45E (RTM), a C₁₄/C₁₅ primary alcohol composition commercially available from Shell Chemical Company, added to which was a solution of 3.8 g of the catalyst slurry in PPG-400, formed by the process described in Example 1, dispersed in 85.1 g of NEODOL 45E, making a total amount of NEODOL 45E of 326.4 g in the reactor. Under constant stirring, the reactor tank was flushed three times with nitrogen, by raising the pressure within the reactor tank to 5 bara and subsequently reducing the pressure to atmospheric pressure. The reactor contents were heated, under a nitrogen atmosphere, to a temperature of 130 °C, and the pressure of nitrogen was increased to approximately 2 bara.
473.4 g of EO was introduced to the reactor, in such a way that the pressure did not exceed 3.6 bara.

After all of the EO had been introduced to the reactor (2.5 hours), the reactor contents were held at the reaction temperature for a further 1 hour, allowing the reaction of residual EO. The reactor contents were then allowed to cool to room temperature.

The total amount of product composition formed was 800 g. The average number of moles of EO per molecule, level of free alcohol and level of 1,4-dioxane were measured using the same methods as used in Example 3. The results are shown in Table 1 below.

The catalyst (solids) intake on total reactor intake was 140 ppm (wt/wt), leading to a concentration of cobalt remaining in the product composition of 15 ppm (wt/wt), and the concentration of zinc remaining in the product composition of 34 ppm (wt/wt).

### Example 6

### DMC catalysed ethoxylation of the secondary alcohol 2-undecanol

2-Undecanol (10.0g) and 0.2g of a 3% slurry of a double metal cyanide catalyst in polypropylene glycol 400 prepared according to Example 1 were stirred in a Schlenk vessel, equipped with a magnetic stirring bar. The vessel was immersed in an oil bath kept at 120°C. Ethylene oxide (EO) was dosed at atmospheric pressure. After an induction period of ca. 3h, EO consumption started. The vessel was weighed several times and after the consumption of 17.9g of EO the reaction was stopped and stripped for about 5 min with nitrogen.
The average number of moles of EO per molecule, level of free alcohol and level of 1,4-dioxane using the same methods as used in Example 3. The results are shown in Table 1 below.

### Example 7

Example 6 was repeated except that before the EO was added, 1ml toluene was added and the mixture stripped with nitrogen at 130°C (to remove water). Then to the remaining reaction mixture (9.3g) EO was added, which reacted immediately. EO dosing was stopped after the consumption of 16.1g.

The average number of moles of EO per molecule, level of free alcohol and level of 1,4-dioxane using the same methods as used in Example 3. The results are shown in Table 1 below.

### Comparative Example A

NEODOL 1-5 (RTM), an ethoxylate derived from a C₁₁ primary alcohol composition and having an average of 5 EO groups per molecule, commercially available from the Shell Chemical Company, prepared using potassium hydroxide as the ethoxylation catalyst, was analysed for average number of moles of EO per molecule, level of free alcohol and level of 1,4-dioxane using the same methods as used in Example 3. The results are shown in Table 1 below.

### Comparative Example B

NEODOL 1-7 (RTM), an ethoxylate derived from a C₁₁ primary alcohol composition and having an average of 7 EO groups per molecule, commercially available from the Shell Chemical Company, prepared using potassium hydroxide as the ethoxylation catalyst, was analysed for average number of moles of EO per molecule, level of free alcohol and level of 1,4-dioxane using the same methods as used in Example 3. The results are shown in Table 1 below.

### Comparative Example C

NEODOL 45-7 (RTM), an ethoxylate derived from a C₁₄/C₁₅ primary alcohol composition and having an average of 7 EO groups per molecule, commercially available from the Shell Chemical Company, prepared using potassium hydroxide as the ethoxylation catalyst, was analysed for average number of moles of EO per molecule, level of free alcohol and level of 1,4-dioxane using the same methods as used in Example 3. The results are shown in Table 1 below.

### Comparative Example D

### Preparation of an ethoxylate derived from a C₁₄/C₁₅ primary alcohol composition and having an average of about 6 EO groups per molecule, produced by acid catalysis

A magnetically stirred PTFE bottle was charged with 22.2 g of NEODOL 45E (RTM), a C₁₄/C₁₅ alcohol composition commercially available from the Shell Chemical Company, 0.5 g of a 10% solution of HF (wt/wt) in NEODOL 45E and 0.05 g of trimethyl borate.

32.6 g of ethylene oxide was bubbled through the solution at such a rate that the bubbles were consumed before reaching the surface. The temperature rapidly increased and was maintained at ± 80°C by external cooling. The reaction was stopped after the ethylene oxide had been consumed and a crystal clear colourless product was obtained.

The ethoxylated C₁₄/C₁₅ alcohol composition prepared made using a HF/borate catalyst was analysed for average number of moles of EO per molecule, level of free alcohol and level of 1,4-dioxane using the same methods as used in Example 3. The results are shown in Table 1 below.

### Comparative Example E

The commercially available NEODOL 91-6 (RTM), an ethoxylate derived from a C₉ - C₁₁ primary alcohol composition and having an average of 6 EO groups per molecule commercially available from the Shell Chemical Company, prepared using potassium hydroxide as the ethoxylation catalyst, was analysed for average number of moles of EO per molecule, level of free alcohol and level of 1,4-dioxane using the same methods as used in Example 3. The results are shown in Table 1 below.

### Comparative Example F

### Potassium hydroxide catalysed ethoxylation of the secondary alcohol 2-undecanol.

2-Undecanol (10.0g) and 0.2g potassium hydroxide were stirred at 130°C. Then 3 ml of toluene were added and removed by stripping with nitrogen (for water removal). To the remaining solution (9.9g), the EO was dosed at atmospheric pressure and stopped after the consumption of 16.7 g of EO. After cooling the mixture was neutralised with acetic acid.

The average number of moles of EO per molecule, level of free alcohol and level of 1,4-dioxane using the same methods as used in Example 3. The results are shown in Table 1 below.

**Table 1**

| | Average Ethoxylation Number | Residual Free Alcohol (/wt.%) | 1,4-Dioxane content (/ppm (wt/wt)) |
|---|---|---|---|
| Example 3. | 5.1 | 1.2 | <5 |
| Example 4. | 6.7 | 0.3 | <5 |
| Example 5. | 7.1 | 0.0 | <10 |
| Example 6 | 7.0 | 0.7 | <5 |
| Example 7 | 6.5 | 1.1 | <10 |
| Comparative Example A. | 5.0 | 4.9 | <5 |
| Comparative Example B. | 7.0 | 2.6 | <5 |
| Comparative Example C. | 7.0 | 2.6 | <5 |
| Comparative Example D. | 5.7 | 1.6 | 22000 |
| Comparative Example E. | 6.0 | 3.9 | <5 |
| Comparative Example F | 6.0 | 5.2 | <10 |

It can be clearly seen from Table 1 (from Examples 3, 4 and 5 and Comparative Examples A, B, C, D and E) that the ethoxylated alcohols prepared from primary alcohols using a DMC catalyst have significantly reduced levels of free alcohol compared to commercially available ethoxylated alcohols prepared from primary alcohols using a conventional potassium hydroxide ethoxylation catalyst. In addition, 1,4-dioxane levels are significantly lower in the ethoxylated alcohols prepared from primary alcohols using a DMC catalyst compared with ethoxylated alcohols prepared using an acid catalyst.

It can also be clearly seen from Table 1 that a DMC catalyst is suitable for the direct ethoxylation of secondary alcohols (Examples 6 and 7) as well as for the ethoxylation of primary alcohols. In the case of secondary alcohols, as is the case for primary alcohols, an ethoxylated alcohol is produced which has significantly reduced levels of free secondary alcohol compared to ethoxylated alcohols prepared using a conventional potassium hydroxide ethoxylation catalyst. In addition, 1,4-dioxane levels are at very low levels in ethoxylated alcohols prepared from secondary alcohols using a DMC catalyst.

In addition to those results shown in Table 1, it was also observed that the ethoxylated alcohols produced using the double metal cyanide catalyst had a more narrow ethylene oxide adduct distribution than the ethoxylated alcohols produced using the conventional potassium hydroxide catalyst.

## Claims

1. Use of a double metal cyanide complex catalyst for reducing the level of free alcohol in an alkoxylate composition prepared by the reaction of an alcohol with an alkylene oxide.

2. Use according to Claim 1 wherein the alcohol is a primary alcohol.

3. Use according to Claim 1 wherein the alcohol is a secondary alcohol.

4. Use according to any of Claims 1 to 3 wherein the level of alcohol in the alkoxylate composition is no more than 2% by weight of composition.

5. Use according to any of Claims 1 to 4 wherein the double metal cyanide catalyst comprises zinc hexacyanocobaltate.

6. Use according to any of Claims 1 to 5 wherein the double metal cyanide catalyst is prepared by a process which comprises the steps of
(a) combining an aqueous solution of a metal salt with an aqueous solution of a metal cyanide salt and reacting these solutions, wherein at least part of this reaction takes place in the presence of an organic complexing agent, thereby forming a dispersion of a solid DMC complex in an aqueous medium;
(b) combining the dispersion obtained in step (a) with an extracting liquid, which is essentially insoluble in water and which is capable of extracting the solid DMC complex formed in step (a) from the aqueous medium, and allowing a two-phase system to be formed consisting of a first aqueous layer and a layer containing the DMC complex and the liquid added;
(c) removing the first aqueous layer; and
(d) recovering the DMC catalyst from the layer containing the DMC catalyst.

7. Use according to Claim 6 wherein the extracting liquid is a compound of general formula (VI) 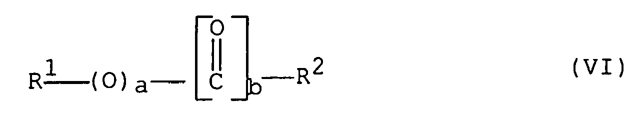 wherein:
R¹ represents hydrogen, an aryl group, a substituted or unsubstituted C1-C10 alkyl group or a group R³-NH,
R² represents hydrogen, an optionally halogenated C1-C10 alkyl group, a group R³-NH-, a group -R⁴-C(O)O-R⁵ or a cyanide group,
R³ represents hydrogen or a C1-C10 alkyl group,
R⁴ represents a substituted or unsubstituted alkylene group having 2 to 15 carbon atoms,
R⁵ represents hydrogen, a substituted or unsubstituted C1-C10 alkyl group, and
a and b independently are 0 or 1.

8. Use according to Claim 7 wherein in the general formula (VI) R¹ and R² independently represent an alkyl group having 1 to 5 carbon atoms, a=1 and b=0.

9. Use according to Claim 7, wherein the compound of general formula (VI) is selected from diethyl ether, methyl tert-butyl ether, di-isopropyl ether and dibutyl ether.

10. Use according to any of Claims 7 to 9 wherein the complexing agent is a water-soluble aliphatic alcohol selected from ethanol, isopropyl alcohol, tert-butyl alcohol, sec-butyl alcohol, n-butyl alcohol and isobutyl, and mixtures thereof.

11. Use according to any of Claims 6 to 10 wherein the double metal cyanide catalyst has the formula (V):
Zn₂[Co(CN)₆]Cl.nC.mH₂O.pA (V)
wherein C is the complexing agent used and A is the compound of general formula (VI) used and n is from 0 to 10, m is from 0 to 20 and p is from 0 to 10.

12. Use according to Claim 11 wherein C is tert-butyl alcohol and A is diethyl ether, methyl tert-butyl ether, di-isopropyl ether and dibutyl ether.

13. Use according to any one of claims 1 to 12, wherein the level of 1,4-dioxane in the alkoxylate composition is also reduced.

14. Use according to claim 1, wherein the alcohol has the formula R'-XH, wherein R' is an alkyl group having from 6 to 30 carbon atoms, X represents an oxygen atom and the alkylene oxide is selected from ethylene oxide, propylene oxide, butylene oxide and mixtures thereof.

15. Use according to claim 1, wherein the alkoxylate composition comprises an alkoxylate having the formula (IV):
R'X(EO)ₓ(PO)_{y}(BO)_{z}H (IV)
wherein R' is an alkyl group having from 6 to 30 carbon atoms, X represents an oxygen, atom, EO represents an ethyleneoxy moiety, PO represents a propyleneoxy moiety, BO represents a butyleneoxy moiety, x is in the range of from 0 to 70, y is in the range of from 0 to 70 and z is in the range of from 0 to 70, and the sum of x, y and z is in the range of from 1 to 70;
wherein the alkoxylate composition comprises no more than 2% by weight of free alcohol having the formula R'-XH where R' and X are as defined above and no more than 10 ppm (wt/wt) of 1,4-dioxane compounds.

## Patentansprüche

1. Verwendung eines Doppelmetallcyanidkomplex-Katalysators zur Verringerung der Menge an freiem Alkohol in einer Alkoxylatzusammensetzung, welche durch die Umsetzung eines Alkohols mit einem Alkylenoxid hergestellt wird.

2. Verwendung nach Anspruch 1, wobei der Alkohol ein primärer Alkohol ist.

3. Verwendung nach Anspruch 1, wobei der Alkohol ein sekundärer Alkohol ist.

4. Verwendung nach einem der Ansprüche 1 bis 3, wobei die Menge an Alkohol in der Alkoxylatzusammensetzung nicht mehr als 2 Gew.% der Zusammensetzung beträgt.

5. Verwendung nach einem der Ansprüche 1 bis 4, wobei der Doppelmetallcyanidkatalysator Zinkhexacyanocobaltat umfaßt.

6. Verwendung nach einem der Ansprüche 1 bis 5, wobei der Doppelmetallcyanidkatalysator durch ein Verfahren hergestellt wird, welches die Schritte von
(a) Kombinieren einer wässerigen Lösung eines Metallsalzes mit einer wässerigen Lösung eines Metallcyanidsalzes und Umsetzen dieser Lösungen, wobei wenigstens ein Teil dieser Reaktion in Gegenwart eines organischen Komplexierungsmittels stattfindet, wodurch eine Dispersion eines festen DMC-Komplexes in einem wässerigen Medium ausgebildet wird;
(b) Kombinieren der, im Schritt (a) erhaltenen Dispersion mit einer Extraktionsflüssigkeit, welche in Wasser im Wesentlichen unlöslich ist und welche fähig ist, den im Schritt (a) ausgebildeten festen DMC-Komplex aus dem wässerigen Medium zu extrahieren, und Ausbildenlassen eines zweiphasigen Systems, welches aus einer ersten wässerigen Schicht und einer den DMC-Komplex und die zugesetzte Flüssigkeit enthaltenden Schicht besteht;
(c) Entfernen der ersten wässerigen Schicht; und
(d) Gewinnen des DMC-Katalysators aus der den DMC-Katalysator enthaltenen Schicht.

7. Verwendung nach Anspruch 6, wobei die Extraktionsflüssigkeit eine Flüssigkeit der allgemeinen Formel (VI) 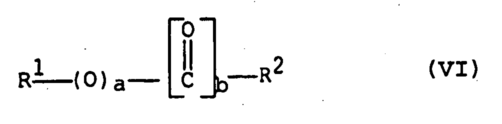 ist, worin
R¹ Wasserstoff, eine Arylgruppe, eine substituierte oder unsubstituierte C₁-C₁₀Alkylgruppe oder eine Gruppe R³-NH darstellt,
R² Wasserstoff, eine wahlweise halogenierte C₁-C₁₀Alkylgruppe, eine Gruppe R³-NH-, eine Gruppe R⁴-C(O)O-R⁵ oder eine Cyanidgruppe darstellt,
R3 Wasserstoff oder eine C₁-C₁₀Alkylgruppe darstellt,
R⁴ eine substituierte oder unsubstituierte Alkylengruppe mit 2 bis 15 Kohlenstoffatomen darstellt,
R⁵ Wasserstoff, eine substituierte oder unsubstituierte C₁-C₁₀Alkylgruppe darstellt, und
a und b unabhängig 0 oder 1 sind.

8. Verwendung nach Anspruch 7, wobei in der allgemeinen Formel (VI) R¹ und R² unabhängig voneinander eine Alkylgruppe mit 1 bis 5 Kohlenstoffatomen darstellen, a 1 ist und b 0 ist.

9. Verwendung nach Anspruch 7, wobei die Verbindung der allgemeinen Formel (VI) unter Diethylether, Methyl-tert.-butylether, Diisopropylether und Dibutylether ausgewählt ist.

10. Verwendung nach einem der Ansprüche 7 bis 9, wobei das Komplexierungsmittel ein wasserlöslicher, aliphatischer Alkohol ist, welcher unter Ethanol, Isopropylalkohol, tert.-Butylalkohol, sec.-Butylalkohol, n-Butylalkohol und Isobutyl, und Gemischen hievon ausgewählt ist.

11. Verwendung nach einem der Ansprüche 6 bis 10, wobei der Doppelmetallcyanidkatalysator die Formel (V)
Zn₂[Co(CN)₆]Cl.nC.mH₂O.pA (V)
besitzt, worin C das verwendete Komplexierungsmittel ist und A die verwendete Verbindung der allgemeinen Formel (VI) ist und n von 0 bis 10 beträgt, m von 0 bis 20 beträgt und p von 0 bis 10 beträgt.

12. Verwendung nach Anspruch 11, wobei C tert.-Butylalkohol darstellt und A Diethylether, Methyl-tert.-butylether, Diisopropylether und Dibutylether darstellt.

13. Verwendung nach einem der Ansprüche 1 bis 12, worin die Menge an 1,4-Dioxan in der Alkoxylatzusammensetzung ebenfalls verringert ist.

14. Verwendung nach Anspruch 1, worin der Alkohol die Formel R'-XH besitzt, worin R' eine Alkylgruppe mit 6 bis 30 Kohlenstoffatomen darstellt, X ein Sauerstoffatom bedeutet und das Alkylenoxid unter Ethylenoxid, Propylenoxid, Butylenoxid und Gemischen hievon ausgewählt ist.

15. Verwendung nach Anspruch 1, wobei die Alkoxylatzusammensetzung ein Alkoxylat der Formel (IV)
R'X(EO)ₓ(PO)_{y}(BO)_{z}H (IV)
umfaßt, worin R' eine Alkylgruppe mit 6 bis 30 Kohlenstoffatomen ist, X ein Sauerstoffatom darstellt, EO einen Ethylenoxyrest darstellt, PO einen Propylenoxyrest darstellt, BO einen Butylenoxyrest darstellt, x im Bereich von 0 bis .70 beträgt, y im Bereich von 0 bis 70 beträgt und z im Bereich von 0 bis 70 beträgt und die Summe von x, y und z im Bereich von 1 bis 70 liegt;
wobei die Alkoxylatzusammensetzung nicht mehr als 2 Gew.-% an freiem Alkohol der Formel R'-XH, worin R' und X wie vorstehend definiert sind, und nicht mehr als 10 ppm (Gewicht/Gewicht) an 1,4-Dioxan-Verbindungen aufweist.

## Revendications

1. Utilisation d'un catalyseur de complexe de cyanure à double métal pour réduire le niveau d'alcool libre dans une composition d'alcoxylate préparée par la réaction d'un alcool avec un oxyde d'alkylène.

2. Utilisation suivant la revendication 1, dans laquelle l'alcool est un alcool primaire.

3. Utilisation suivant la revendication 1, dans laquelle l'alcool est un alcool secondaire.

4. Utilisation suivant l'une quelconque des revendications 1 à 3, dans laquelle le niveau d'alcool dans la composition d'alcoxylate n'est pas plus de 2 % en poids de la composition.

5. Utilisation suivant l'une quelconque des revendications 1 à 4, dans laquelle le catalyseur de cyanure à double métal comprend de l'hexacyanocobaltate de zinc.

6. Utilisation suivant l'une quelconque des revendications 1 à 5, dans laquelle le catalyseur de cyanure à double métal est préparé par un procédé qui comprend les étapes de :
(a) combinaison d'une solution aqueuse d'un sel métallique avec une solution aqueuse d'un sel de cyanure métallique et réaction de ces solutions, dans laquelle au moins une partie de cette réaction se fait en présence d'un agent complexant organique, en formant ainsi une dispersion d'un complexe de DMC solide dans un milieu aqueux;
(b) combinaison de la dispersion obtenue dans l'étape (a) avec un liquide d'extraction, qui est essentiellement insoluble dans l'eau et qui peut extraire le complexe de DMC solide formé dans l'étape (a) du milieu aqueux, et formation d'un système à deux phases composé d'une première couche aqueuse et d'une couche contenant le complexe de DMC et le liquide ajouté;
(c) enlèvement de la première couche aqueuse; et
(d) récupération du catalyseur de DMC de la couche contenant le catalyseur de DMC.

7. Utilisation suivant la revendication 6, dans laquelle le liquide d'extraction est un composé de formule générale (VI) : 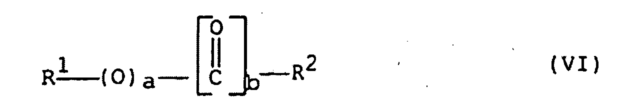 dans laquelle:
R¹ représente de l'hydrogène, un groupe aryle, un groupe alkyle en C1-C10 substitué ou non substitué ou un groupe R³-NH,
R² représente de l'hydrogène, un groupe alkyle en C1-C10 éventuellement halogéné, un groupe R³-NH-, un groupe -R⁴-C(O)O-R⁵ ou un groupe cyanure,
R³ représente de l'hydrogène ou un groupe alkyle en C1-C10,
R⁴ représente un groupe alkylène substitué ou non substitué comportant 2 à 15 atomes de carbone,
R⁵ représente de l'hydrogène, un groupe alkyle en C1-C10 substitué ou non substitué, et
a et b sont indépendamment 0 ou 1.

8. Utilisation suivant la revendication 7, dans laquelle dans la formule générale (VI) R¹ et R² représentent indépendamment un groupe alkyle comportant 1 à 5 atomes de carbone, a=1 et b=0.

9. Utilisation suivant la revendication 7, dans laquelle le composé de formule générale (VI) est choisi parmi l'éther diéthylique, l'éther méthyl tert-butylique, l'éther diisopropylique et l'éther dibutylique.

10. Utilisation suivant l'une quelconque des revendications 7 à 9, dans laquelle l'agent complexant est un alcool aliphatique soluble dans l'eau choisi parmi l'éthanol, l'alcool isopropylique, l'alcool tert-butylique, l'alcool sec-butylique, l'alcool n-butylique et l'alcool isobutylique, et leurs mélanges.

11. Utilisation suivant l'une quelconque des revendications 6 à 10, dans laquelle le catalyseur de cyanure à double métal a la formule (V) :
Zn₂[Co(CN)₆]Cl.nC.mH₂O.pA (V)
dans laquelle C est l'agent complexant utilisé et A est le composé de formule générale (VI) utilisé et n vaut de 0 à 10, m vaut de 0 à 20 et p vaut de 0 à 10.

12. Utilisation suivant la revendication 11, dans laquelle C est de l'alcool tert-butylique et A est de l'éther diéthylique, de l'éther méthyl tert-butylique, de l'éther diisopropylique ou de l'éther dibutylique.

13. Utilisation suivant l'une quelconque des revendications 1 à 12, dans laquelle le niveau de 1,4-dioxanne dans la composition d'alcoxylate est également réduit.

14. Utilisation suivant la revendication 1, dans laquelle l'alcool a la formule R'-XH, dans laquelle R' est un groupe alkyle comportant de 6 à 30 atomes de carbone, X représente un atome d'oxygène et l'oxyde d'alkylène est choisi parmi l'oxyde d'éthylène, l'oxyde de propylène, l'oxyde de butylène et leurs mélanges.

15. Utilisation suivant la revendication 1, dans laquelle la composition d'alcoxylate comprend un alcoxylate ayant la formule (IV) :
R'X(EO)ₓ(PO)_{y}(BO)_{z}H (IV)
dans laquelle R' est un groupe alkyle comportant de 6 à 30 atomes de carbone, X représente un atome d'oxygène, EO représente un fragment éthylénoxy, PO représente un fragment propylénoxy, BO représente un fragment butylénoxy, x se situe dans la plage de 0 à 70, y se situe dans la plage de 0 à 70 et z se situe dans la plage de 0 à 70, et la somme de x, y et z se situe dans la plage de 1 à 70;
dans laquelle la composition d'alcoxylate ne comprend pas plus de 2 % en poids d'alcool libre ayant la formule R'-XH dans laquelle R' et X sont tels que définis précédemment et pas plus de 10 ppm (poids/poids) de composés de 1,4-dioxanne.
